# EUROPEAN PATENT APPLICATION

(11) **EP 4 825 434 A1**
(43) Date of publication of application: **21.10.2026**
(21) Application number: 26172897.6
(22) Date of filing: 16.04.2026
(51) Int. Cl.: A61N 1/05

(54) **IMPLANTABLE LEAD TORQUE TRANSFER TOOL**

(30) Priority: 18.04.2025 US 202563791320 P; 15.04.2026 US 202619648869
(71) Applicant: Pacesetter, Inc., Sylmar, CA 91342 (US)
(72) Inventor: FRANZ, Claire, Sylmar, CA 91342 (US); ALLEMAN, Wesley, Sylmar, CA 91342 (US); LEDBETTER, Cody, Sylmar, CA 91342 (US); FONG, Peter, Sylmar, CA 91342 (US); VICTORINE, Keith, Sylmar, CA 91342 (US)
(74) Representative: Barker Brettell Sweden AB

(57) **Abstract**

An implantable lead (100) includes a lead body (202) extending along a central axis (204) and having a central lumen (208). The implantable lead (100) includes a drive coupling (310) rotatably mounted in the central lumen (208). The drive coupling (310) includes a drive shaft (314) having an outer shaft surface (402) defining a non-circular shaft profile (602). The implantable lead (100) includes a helical fixation element (108) mounted on the drive coupling (310).

## Description

### TECHNICAL FIELD

The present disclosure relates to implantable leads. More specifically, the present disclosure relates to implantable leads useful for septal pacing.

### BACKGROUND INFORMATION

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Conduction system pacing (CSP) can use implantable leads, such as pacing or defibrillation leads, to provide an electrical connection between the artificial pacemaker and a target location of the cardiac conduction system that is to be electrically stimulated. The target anatomy may be, for example, a His bundle of a heart. Cardiac pacing of the His bundle is clinically effective and advantageous by providing a narrow QRS affecting synchronous contraction of the ventricles. His bundle pacing in or near a membranous septum of a heart, however, has some drawbacks. Successful His bundle pacing cannot always be achieved. Pacing thresholds are often high, sensing is challenging, and success rates can be low.

CSP at the left bundle branch (LBB) is an alternative to His bundle pacing. Pacing at the LBB involves pacing past the His bundle toward the right ventricle apex. More particularly, a pacing site for LBB pacing is typically below the His bundle, on the interventricular septal wall. To achieve optimal results, the pacing site for physiological pacing at the LBB can be relatively deep in the interventricular septal wall, compared to the His bundle.

### SUMMARY

Existing approaches for conduction system pacing (CSP) use existing implantable leads, such as pacing or defibrillation leads, which include a fixed or extendable helix to engage the target tissue. The target pacing site can be deep in the interventricular septal wall. For example, the target pacing site of left bundle branch (LBB) area pacing may be several centimeters deep in the interventricular septal wall. Therefore, a physician may be required to rotate and push an implantable lead to drive the implantable lead through the myocardial tissue to the target pacing site. Existing implantable leads are flexible by design, however, and the application of torque to a proximal end of the implantable lead can cause deformation, e.g., wrinkling, and torque build up over a lead length. Such deformation can be exacerbated by variations in tissue consistency, which can complicate the procedure for physicians. More particularly, the deformation and torque build up can stress lead components and interfere with lead placement. Thus, there is a need for an implantable lead or implantable lead system that efficiently transfers torque input at the proximal end by the physician to burrow a distal end of the implantable lead through the interventricular septal wall to the target tissue. Efficient torque transfer could make implantation more predictable, regardless of the tissue consistency being encountered by the implantable lead.

An implantable lead is described. In an embodiment, the implantable lead includes a lead body extending along a central axis and having a central lumen. The implantable lead includes a drive coupling rotatably mounted in the central lumen. The drive coupling includes a drive shaft having an outer shaft surface defining a shaft profile. The shaft profile is non-circular. The implantable lead includes a helical fixation element mounted on the drive coupling.

A drive stylet is described. In an embodiment, the drive stylet includes a stylet body extending along a central axis from a proximal stylet portion to a distal stylet portion. The stylet body includes a stylet tip at the distal stylet portion. The stylet tip includes an outer tip surface defining a tip profile. The tip profile is non-circular. The drive stylet includes a knob mounted on the proximal stylet portion of the stylet body.

An implantable lead system is described. The implantable lead system includes the implantable lead and the drive stylet.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross-sectional view of a patient heart illustrating an example implantation of an implantable lead in a target anatomy, in accordance with an embodiment.
FIG. 2 is a perspective view of an implantable lead, in accordance with an embodiment.
FIG. 3 is a cross-sectional view of an implantable lead, in accordance with an embodiment.
FIG. 4 is a cutaway, perspective view of a drive mechanism of an implantable lead system, in accordance with an embodiment.
FIG. 5 is a perspective view of a drive coupling and helical fixation element of an implantable lead, in accordance with an embodiment.
FIG. 6 is a sectional view of a drive coupling of an implantable lead, in accordance with an embodiment.
FIG. 7 is a perspective view of a drive coupling of an implantable lead, in accordance with an embodiment.
FIG. 8 is a side view of a distal stylet portion of a drive stylet, in accordance with an embodiment.
FIG. 9 is a perspective view of a distal stylet portion of a drive stylet, in accordance with an embodiment.
FIG. 10 is a sectional view of a stylet tip of a drive stylet, in accordance with an embodiment.
FIG. 11 is a sectional view of a drive stylet engaging a drive coupling of an implantable lead, in accordance with an embodiment.
FIG. 12 is a perspective view of an implantable lead system having a drive stylet inserting into an implantable lead, in accordance with an embodiment.
FIG. 13 is a sectional view of an implantable lead system having a drive stylet disengaged from a drive coupling of an implantable lead, in accordance with an embodiment.
FIG. 14 is a perspective view of a locking tool on a drive stylet of an implantable lead system, in accordance with an embodiment.
FIG. 15 is a perspective view of a locking tool holding an implantable lead and a drive stylet of an implantable lead system, in accordance with an embodiment.
FIG. 16 is a sectional view of an implantable lead system having a drive stylet engaged with a drive coupling of an implantable lead, in accordance with an embodiment.
FIG. 17 is a perspective view of a locking tool rotating an implantable lead system, in accordance with an embodiment.

### DETAILED DESCRIPTION

Embodiments describe an implantable lead, e.g., a pacing or defibrillation lead, for cardiac stimulation, e.g., pacing. The implantable lead may, however, be used in other applications, such as deep brain stimulation. Thus, reference to the implantable lead as being used for cardiac, e.g., septal, stimulation is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of an implantable lead, a drive stylet, or an implantable lead system. Similarly, "proximal" may indicate a second direction opposite to the first direction. Such terms are provided to establish relative frames of reference, however, and are not intended to limit the use or orientation of the implantable lead, the drive stylet, or the implantable lead system to a specific configuration described in the various embodiments below.

In an aspect, an implantable lead includes a drive coupling. The drive coupling includes a shaft profile that is non-circular. In an embodiment, a stylet tip of a drive stylet, which is also non-circular, can engage the drive coupling to deliver torque to a distal end of the implantable lead. For example, the non-circular profiles of the drive coupling and the drive stylet can be complementary and can engage to transmit torque directly to the drive coupling. Accordingly, a helical fixation element of the implantable lead can be efficiently and reliably driven to deliver, e.g., burrow, the implantable lead through the interventricular wall to a target pacing site for CSP, regardless of a makeup of the interventricular wall tissue.

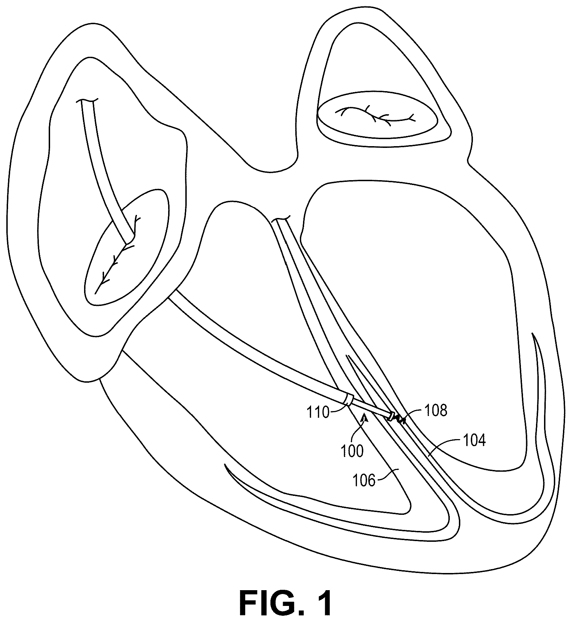

Referring to FIG. 1, a diagrammatic cross-sectional view of a patient heart illustrating an example implantation of an implantable lead in a target anatomy is shown in accordance with an embodiment. An implantable lead 100, e.g., a pacing lead or an implantable cardioverter defibrillator lead, may be a transvenous lead that can be delivered to a target anatomy to conductively pace the target anatomy. The target anatomy can include a septal wall 106 of a heart and/or a LBB 104 in the septal wall 106.

The implantable lead 100 may be any type of pacing lead. For example, the implantable lead 100 can include an extendable or retractable helix, or a fixed helix. Furthermore, the implantable lead 100 may be unipolar or bipolar. The implantable lead 100 may be of a high voltage or a low voltage lead type. Accordingly, the illustrations and descriptions with respect to lead structure are provided herein by way of example and not limitation.

In an embodiment, the implantable lead 100 can include an active fixation element, e.g., a helical fixation element 108. The helical fixation element 108 can include an electrode, which may be fixed or extendable, relative to a ring electrode 110. The implantable lead 100 can be driven into the interventricular septum to engage and anchor the implantable lead 100 to the tissue. The helical fixation element 108 can be an active electrode, and thus, can pace the tissue. When the helical fixation element 108 is located at the LBB 104, the ring electrode 110 can be located at or on a surface of the septal wall 106. The ring electrode 110 can be a passive or an active electrode. Accordingly, the electrodes can facilitate optimal placement of each electrode to provide a more robust multisite pacing modality.

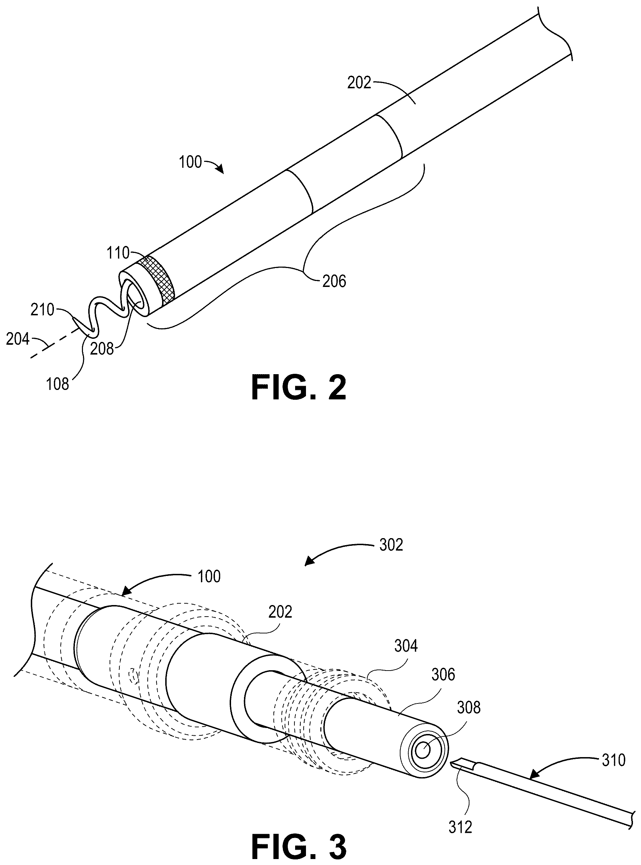

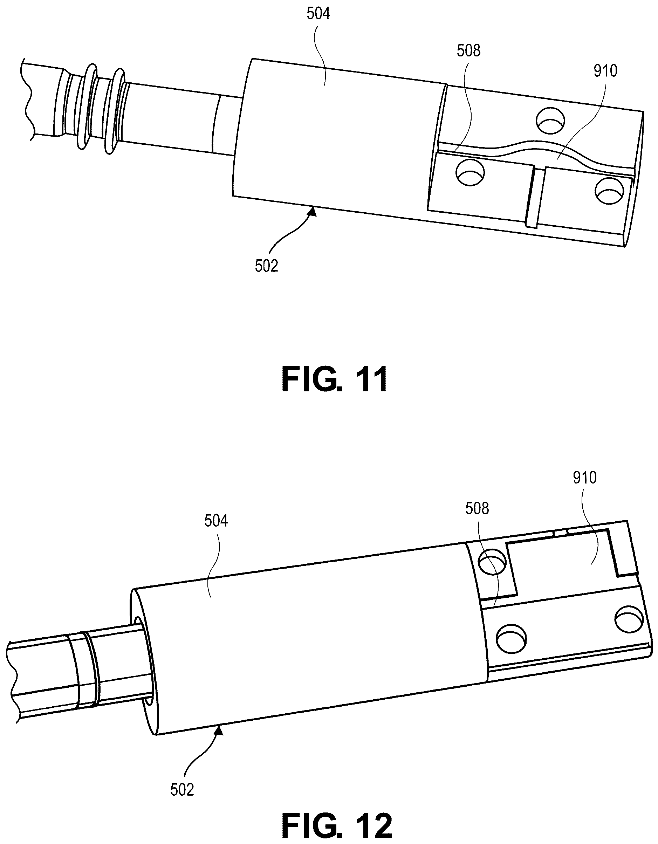

Referring to FIG. 2, a perspective view of an implantable lead is shown in accordance with an embodiment. The implantable lead 100 can include a lead body 202 extending along a central axis 204 from a proximal lead portion (FIG. 12) to a distal lead portion 206. The lead body 202 can include a central lumen 208 in the distal lead portion 206. The central lumen 208 can house a portion of the helical fixation element 108. More particularly, the helical fixation element 108 can extend distally from the central lumen 208. In an embodiment, the helical fixation element 108 is fixed, with a distal tip 210 permanently extended beyond an end of the lead body 202. In an embodiment, the helical fixation element 108 is extendable, with the distal tip 210 capable of extending or retracting from the central lumen 208, or relative to a distal end of the lead body 202.

As described above, the implantable lead 100 can have one or more electrodes, such as the helical fixation element 108 and/or the ring electrode 110. The electrode(s) can be driven into target tissue or pacing. For example, the distal tip 210 of the helical fixation element 108 can be screwed into the interventricular septal wall 106 to contact and pace a LBB 104.

Referring to FIG. 3, a cross-sectional view of an implantable lead system 300 is shown in accordance with an embodiment. The implantable lead system 300 includes the implantable lead 100 and a drive stylet 301. The drive stylet 301 can engage the implantable lead 100 to transfer torque and drive the implantable lead 100 into the target tissue.

The lead body 202 can be further divided into individual components. In an embodiment, the lead body 202 includes an outer jacket 302 having a tubular, elongate structure. The outer jacket 302 may, for example, be an insulating jacket surrounding an outer coil (not shown). More particularly, the outer coil can include a coil extending from a proximal portion of the implantable lead 100 (FIG. 12) to a distal lead portion 206 of the implantable lead 100 (shown in cross-section in FIG. 3), and the outer jacket 302 can be a silicone sleeve or layer mounted on the outer coil. The outer coil may be formed from an electrically conductive material to conduct sensing or stimulation, e.g., pacing, pulses from an external stimulation, e.g., pacing, device to the ring electrode 110. Accordingly, the outer jacket 302 can insulate the outer coil from a surrounding environment over a length that the outer jacket 302 covers the outer coil. In combination, the outer jacket 302 and the outer coil can form the lead body 202 having the central lumen 208 through which an inner lead subassembly extends.

The inner lead subassembly can include a tubular, elongate structure. For example, the inner lead subassembly can have an inner coil 306 extending helically about, and longitudinally along, the central axis 204. More particularly, the inner coil 306 can extend through the lead body 202 along the central axis 204, e.g., within the central lumen 208. The inner coil 306 may be formed from an electrically conductive material, and thus, can conduct electrical stimulation pulses from the external stimulation device to distal structures that the inner coil 306 electrically communicates with, e.g., the helical fixation element 108.

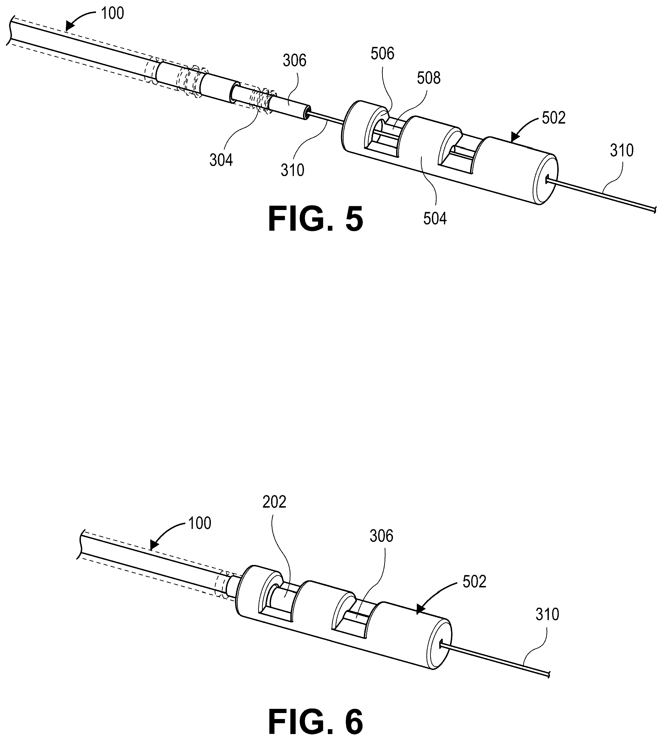

The implantable lead 100 can include a drive coupling 310. The drive coupling 310 can have a shaft structure, e.g., a rotationally symmetric structure, including lengths to engage surrounding structures. For example, the drive coupling 310 can include a distal boss, e.g., a mounting prong (FIG. 5), to engage the helical fixation element 108. More particularly, the helical fixation element 108 can have a helix lumen extending through the helical coil (the helical coil can extend about the helix lumen) and the helical fixation element 108 can be mounted on the drive coupling 310 such that the distal boss is disposed within the helix lumen.

The drive coupling 310 may also be coupled to the inner coil 306. For example, the inner coil 306 can have a coil lumen 312, and a proximal boss, e.g., a mounting segment (FIG. 5) of the drive coupling 310 can extend into the coil lumen 312. More particularly, the drive coupling 310 can be disposed in the coil lumen 312 and connected to the inner coil 306 to secure the inner coil 306 to the drive coupling 310. The connection may be via an adhesive or thermal joint or weld, for example, as described below.

Continuing to refer to FIG. 3, via the physical connections described above, the inner coil 306 can be electrically connected to the drive coupling 310 and the helical fixation element 108 can be electrically connected to the drive coupling 310. The structures may be conductive and, thus, pacing and sensing pulses may be communicated between the helical fixation element 108 and the inner coil 306 via the drive coupling 310.

The drive coupling 310 may be fixed or rotatable relative to the lead body 202. In an embodiment, the drive coupling 310 is fixed. For example, the drive coupling 310 can have an outer surface that is interconnected with the outer jacket 302 of the implantable lead 100. Accordingly, when torque is applied to the outer jacket 302 or the inner coil 306, such torque can be transmitted to the drive coupling 310. As described below, torque may also be delivered directly to the drive coupling 310, e.g., by engagement between mating drive surfaces of the drive coupling 310 and the drive stylet 301. The interconnection may be via an adhesive or thermal joint or weld, for example. Alternatively, the lead body 202, or a portion thereof, may be overmolded or heat shrunk onto the drive coupling 310.

In an embodiment, the drive coupling 310 is rotatable relative to the lead body 202. The drive coupling 310 may be rotatably mounted in the central lumen 208. For example, the drive coupling 310 can have an outer surface that is seated within or against a collar 315 of the lead body 202. The outer surface can rotatably slide within or against the collar 315 about the central axis 204 when torque is applied to the drive coupling 310. Such torque can be applied to the drive coupling 310 by the inner coil 306 or the drive stylet 301. In an embodiment, the inner coil 306 is coupled to the drive coupling 310. For example, the drive coupling 310 may be disposed in a coil lumen 312 of the inner coil 306, and the inner coil 306 can be attached to the outer surface of the drive coupling 310. The attachment between the inner coil 306 and the drive coupling 310 can transmit torque from the coil to the coupling. When the inner coil 306 is rotated within the central lumen 208 of the lead body 202, the rotation is transmitted to the drive coupling 310.

In an embodiment, torque can be transmitted to the drive coupling 310 from a proximal end of the implantable lead 100 via the drive stylet 301. The drive coupling 310 can include a drive shaft 314 to receive a torque input from the drive stylet 301. More particularly, the drive stylet 301 can, as described below, include an outer tip surface to engage an outer shaft surface of the drive shaft 314. The outer shaft surface can be the outermost radial surface of the drive shaft 314, facing outward away from an interior portion of the shaft at the location where the profile is being determined, e.g., by cross-sectional analysis. Accordingly, the outer tip surface of the drive stylet 301 engages the drive shaft 314 at an outward-facing surface, rather than through insertion of a drive tip into an inward-facing slot or recess in the interior of the drive shaft 314. The drive stylet 301 can be rotated to torque the drive shaft 314 and rotate the drive coupling 310. Accordingly, the drive coupling 310 can torque the helical fixation element 108 and/or the lead body 202 to burrow the implantable lead 100 into the target tissue.

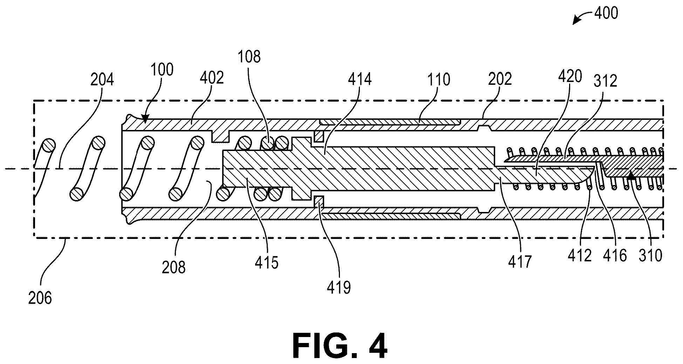

Referring to FIG. 4, a cutaway, perspective view of a drive mechanism of an implantable lead system 300 is shown in accordance with an embodiment. The drive coupling 310, which is positioned along the central axis 204 within the lead body 202, can have a shape to interface with adjacent components. In an embodiment, the drive coupling 310 includes the drive shaft 314, which can interface with the inner coil 306 and a drive stylet 301. The drive shaft 314 has an outer shaft surface 402, which can have varying sectional profiles along a length. For example, a distal sectional profile 411, taken near a step surface of the drive coupling 310, may be circular. By contrast, a proximal sectional profile 413, referred to as a shaft profile below, may be non-circular. The outer shaft surface 402 can be the outermost radial surface of the drive shaft 314. As opposed to, for example, an internal slot within a cylindrical shaft, which would be defined by an interior surface radially inward from an outermost radial surface of the cylindrical shaft, the outer shaft surface 402 can be the outermost radial surface when the component is viewed in a direction perpendicular to the central axis 204. Accordingly, engagement between the drive stylet 301 and the drive shaft 314 is engagement between the outward-facing outer tip surface of the drive stylet 301 and the outward-facing outer shaft surface 402 of the drive shaft 314, and no insertion of a drive tip into an interior slot or cavity is required to achieve torque transmission.

The circular profile of the drive shaft 314, can interface with the inner coil 306, effectively filling the coil lumen 312 and providing a round surface upon which the inner coil 306 may be mounted. By contrast, the non-circular profile creates a complementary space 406, which is disposed between a shaft drive surface 408 of the drive shaft 314 and an inner coil surface 410 of the inner coil 306. The complementary space 406 can receive the drive stylet 301 to allow the drive stylet 301 to transfer torque to the drive coupling 310, as described below.

In an embodiment, the inner coil 306 is fixed to the drive shaft 314. For example, a joint 420 may be disposed between the inner coil 306 and the drive shaft 314. The joint 420 may be an adhesive or thermal joint, such as a weld, which connects a distal coil end of the inner coil 306 to a distal section of the drive shaft 314. The joint 420 may be between the distal coil end and a stepped surface 404 of the drive shaft 314.

The outer shaft surface 402 of the drive shaft 314 can include the stepped surface 404, which may be a transverse surface that extends radially outward from the drive shaft 314 to a coupling body 424 of the drive coupling 310. More particularly, the coupling body 424 can be distal to the drive shaft 314 and have a larger transverse dimension than the drive shaft 314 and, thus, the stepped surface 404 extending between the drive shaft 314 and the coupling body 424 can extend radially outward. The transverse surface can form a corner within which the material, e.g., adhesive or reflowed metal, of the joint 420 can flow and connect the inner coil 306 to the drive shaft 314. The joint 420 can mechanically and electrically interconnect the inner coil 306 to the drive coupling 310.

Referring to FIG. 5, a perspective view of a drive coupling 310 and helical fixation element 108 of an implantable lead 100 is shown in accordance with an embodiment. Additional sections of the drive coupling 310 are shown along the length of the drive coupling 310. At the proximal end, the drive shaft 314 having the non-circular profile is shown, in which the shaft drive surface 408 extends between a first shaft edge 502 and a second shaft edge 504, as described below. Distal to the non-circular profile is a mounting segment 506 on which the inner coil 306 is mounted and joined to the stepped surface 404. The coupling body 424 extends distally from the drive shaft 314, to a collar 508. In an embodiment, the collar 508 has a larger diameter than the coupling body 424, and provides a backstop for the helical fixation element 108. More particularly, a proximal end of the helical fixation element 108 can abut the collar 508. A mounting prong 510 may extend distally from the collar 508, into an inner lumen of the helical fixation element 108. More particularly, the helical fixation element 108 can be mounted on, and extend around, the mounting prong 510. The helical fixation element 108 may be joined, e.g., by a weld, to the mounting prong 510 or the collar 508.

Referring to FIG. 6, a sectional view of a drive coupling 310 of an implantable lead 100 is shown in accordance with an embodiment. The sectional view is taken through the portion of the non-circular portion of the drive shaft 314, proximal to the circular portion on which the inner coil 306 is mounted. The cross-section reveals a shaft profile 602, defined by the line extending around the drive shaft 314 along the outer shaft surface 402. As shown, the shaft profile 602 is non-circular, i.e., the profile line is not a circle. Because the shaft profile 602 is defined by the outermost radial surface of the drive shaft 314, the non-circular shape is an outward-facing profile available for engagement with a complementary outward-facing surface of the drive stylet 301, distinguishing it from a non-circular profile defined by an inward-facing interior surface, such as a slot or socket machined into the interior of a cylindrical shaft.

In an embodiment, the shaft profile 602 is formed by removing a portion of the circular shaft during manufacturing, e.g., by milling or molding the non-circular profile into the drive shaft 314. Accordingly, the shaft profile 602 has a shaft circular arc 604, which may be a portion of the originally cylindrical shaft that was not removed. The shaft circular arc 604 can extend about the central axis 204 between the first shaft edge 502 and the second shaft edge 504.

The shaft profile 602 can include another profile line extending between the first shaft edge 502 and the second shaft edge 504, which combines with the shaft circular arc 604 to surround the sectional area of the drive shaft 314. In an embodiment, additional profile line can be non-circular. For example, the shaft profile 602 include a shaft non-circular segment 606 extending between the first shaft edge 502 and the second shaft edge 504. The shaft non-circular segment 606 may be linear, e.g., straight, or curvilinear. However, the shaft non-circular segment 606 may have a different radius than the shaft circular arc 604. Accordingly, the shaft circular arc 604 and the shaft non-circular segment 606 do not combine to form a circle.

In an embodiment, the transverse cross-sectional area of the drive shaft 314, at a location on the shaft drive surface 408, provides a "D" interface. More particularly, the shaft profile 602 can include a semi-circular segment 608. The semicircular segment 608 may be formed by the shaft circular arc 604, which may be an arc having a radius of the drive shaft 314 and spanning 180 degrees between the first shaft edge 502 and the second shaft edge 504, and a straight shaft non-circular segment 606, extending between the shaft edges 502, 504 and through (or immediately adjacent to, accounting for a slip fit gap between the adjacent torque transmission components) the central axis 204. The semi-circular segment 608 forms a "D" profile, which can interface with the drive stylet 301 having a similar "D" profile, as described below, to form a mated pair. Because the "D" profile is defined by the outward-facing outer shaft surface 402, engagement with the drive stylet 301 occurs at the outer surface of the drive shaft 314, rather than requiring the drive stylet 301 to be received within an inward-facing interior slot or recess of the drive shaft 314. The embodiments are, however, not limited to a "D" profile and also other non-circular profiles could be used for the non-circular segment 608.

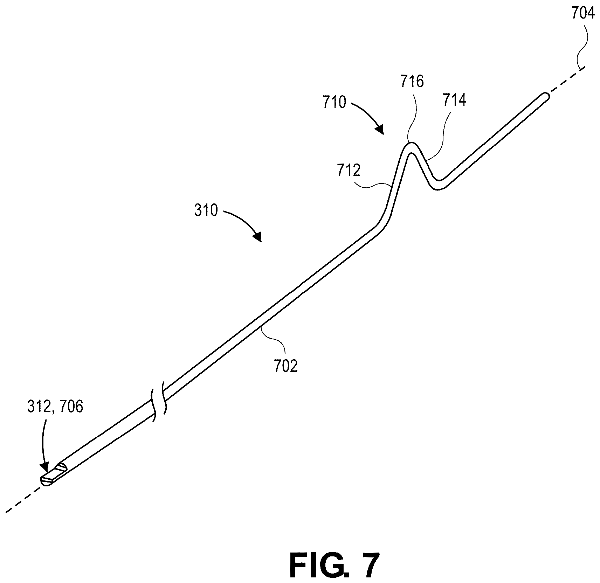

Referring to FIG. 7, a perspective view of a drive coupling 310 of an implantable lead 100 is shown in accordance with an embodiment. The drive shaft 314 interface is shown in detail to illustrate the "D" interface in perspective and to show a lead in feature that facilitates smooth engagement between the drive shaft 314 and the drive stylet 301. The lead in feature permits the drive stylet 301, when it is inserted through the central lumen 208 of the inner coil 306, to slide into place within the complementary space 406 between the shaft drive surface 408 and the inner coil surface 410.

In an embodiment, the lead in feature of the drive shaft 314 may include a transitional edge 702 between the shaft drive surface 408 and a proximalmost surface of the drive shaft 314. The proximalmost surface can be at a proximal shaft end 704. Accordingly, the transitional edge 702 eases the transition between the proximal shaft end 704 and the shaft drive surface 408. For example, the transitional edge 702 can include a chamfer, a fillet, or another lead in edge that allows the drive stylet 301 to slide into engagement with the drive shaft 314.

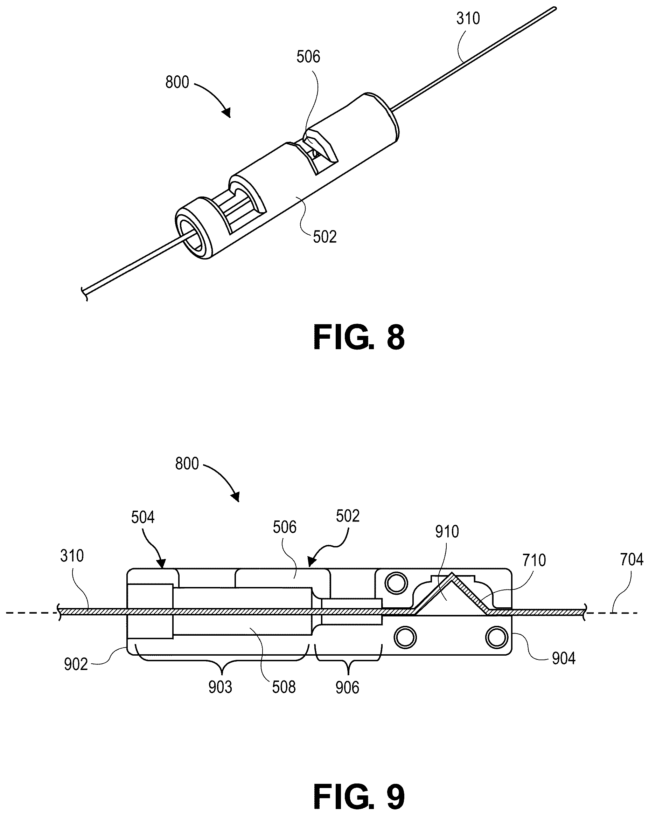

Referring to FIG. 8, a side view of a distal stylet portion 806 of a drive stylet 301 is shown in accordance with an embodiment. The drive stylet 301 can include a similar "D" interface as the drive shaft 314 to allow the drive stylet 301 to engage the drive shaft 314 and directly transfer torque to the tissue through the engaged components. In an embodiment, the drive stylet 301 includes a stylet body 805 extending along the central axis 204. For example, the stylet body 805 can extend from a proximal stylet portion 804 to a distal stylet portion 806 over a length of the drive stylet 301. The stylet body 805 may, for example, be an elongated wire extending between the proximal and distal portions 804, 806 to a stylet tip 808 at the distal stylet portion 806. As described below, the stylet tip 808 can engage the drive shaft 314, allowing torque to be transferred directly to the drive coupling 310 through the elongated stylet body 805 and eliminating the need to transfer torque through the lead body 202. Torque transfer efficiency is thereby improved to make burrowing the implantable lead 100 into the septal wall 106 easier and more predictable for the physician.

Referring to FIG. 9, a perspective view of a distal stylet portion 806 of a drive stylet 301 is shown, in accordance with an embodiment. The stylet tip 808 of the drive stylet 301 can include an outer tip surface 902. The outer tip surface 902 defines a shape of the stylet tip 808 and, more particularly, defines a tip profile 904. The outer tip surface 902 is the outermost radial surface of the stylet tip 808, facing outward away from the central axis 204, and is thus configured to engage the outward-facing outer shaft surface 402 of the drive shaft 314. The tip profile 904 can, like the shaft profile 602, include a perimeter of a cross-sectional area of the stylet tip 808 taken at a location along a tip drive surface 906. The tip profile 904 can have a shape that is complementary to the shaft profile 602, as described below. Furthermore, like the drive shaft 314, the drive stylet 301 may have a lead in edge 950 at a proximal end to facilitate smooth engagement of the intermeshing portions of the components when the tip drive surface 906 is advanced onto the shaft drive surface 408.

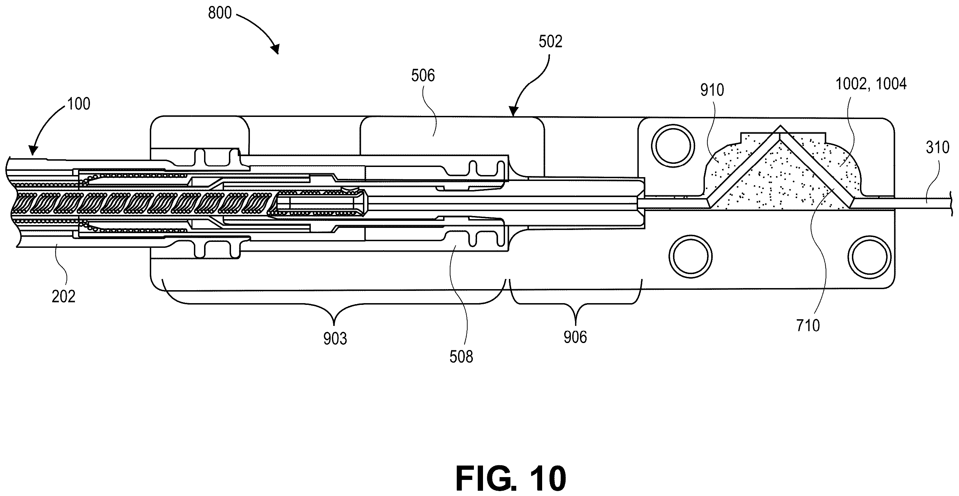

Referring to FIG. 10, a sectional view of a stylet tip 808 of a drive stylet 301 is shown in accordance with an embodiment. The sectional view is taken through the stylet tip 808 at a location along the tip drive surface 906. The sectional area shown may be distal to a circular portion of the drive stylet 301 that extends along the proximal stylet portion 804. The cross-section reveals the tip profile 904, defined by the line extending around the drive stylet 301 along the outer tip surface 902. As shown, the tip profile 904 is non-circular, i.e., the profile line is not a circle. The non-circular tip profile 904 is defined by the outward-facing outer tip surface 902, and is therefore configured for outward-surface engagement with the non-circular shaft profile 602 of the drive shaft 314, rather than for insertion into an inward-facing interior slot or recess of a cylindrical component.

In an embodiment, the tip profile 904 is formed by removing a portion of the circular shaft during manufacturing, e.g., by milling or molding the non-circular profile into the drive stylet 301. Accordingly, the tip profile 904 has a tip circular arc 1006, which may be a portion of the originally cylindrical shaft that was not removed. The tip circular arc 1006 can extend about the central axis 204 between a first tip edge 1002 and a second tip edge 1004. As shown in FIG. 9, the tip edges 1002, 1004 can extend longitudinally on either side of the tip drive surface 906.

The tip profile 904 can include another profile line extending between the first tip edge 1002 and the second tip edge 1004, which combines with the tip circular arc 1006 to surround the sectional area of the drive stylet 301. In an embodiment, the additional profile line can be non-circular. For example, the tip profile 904 include a tip non-circular segment 1008 extending between the first tip edge 1002 and the second tip edge 1004. The tip non-circular segment 1008 may be linear, e.g., straight, or curvilinear. However, the tip non-circular segment 1008 may have a different radius than the tip circular arc 1006. Accordingly, the tip circular arc 1006 and the tip non-circular segment 1008 do not combine to form a circle.

In an embodiment, the transverse cross-sectional area of the drive stylet 301, at a location on the tip drive surface 906, provides a "D" interface. More particularly, the tip profile 904 can include a semi-circular segment 1005. The semi-circular segment 1005 may be formed by the tip circular arc 1006, which may be an arc having a radius of the drive stylet 301 and spanning 180 degrees between the first tip edge 1002 and the second tip edge 1004, and a straight tip non-circular segment 1008, extending between the tip edges 1002, 1004 and through (or immediately adjacent to) the central axis 204. The semi-circular segment 1005 forms a "D" profile, which can interface with the drive coupling 310 have the similar "D" profile, as described above, to form a mated pair.

Referring to FIG. 11, a sectional view of a drive stylet 301 engaging a drive coupling 310 of an implantable lead 100 is shown in accordance with an embodiment. The sectional view shows the stylet tip 808 engaged with the drive shaft 314. When the stylet tip 808 engages the drive shaft 314, the outer shaft surface 402 and the outer tip surface 902 may be complementary to form a circular outer profile 1102. More particularly, the shaft circular arc 604 and the tip circular arc 1006, both of which may be "D" shaped and/or semi-circular, can combine to form a full circle. The circular outer profile 1102 can conform to the inner coil 306. For example, the inner coil 306 can have a cylindrical coil lumen 312, and the combined drive shaft 314 and stylet tip 808 can fill the lumen. Accordingly, filars of the inner coil 306 can surround and support the interconnection between the drive coupling 310 and the drive stylet 301, keeping the stylet tip 808 engaged with the drive shaft 314. This configuration, in which the non-circular shaft profile 602 and tip profile 904 are each defined by outward-facing surfaces that engage one another, enables torque transmission through outward-surface contact, in contrast to a drive configuration in which a drive tip is received within an inward-facing slot or interior cavity of a cylindrical component.

The sectional profiles of the drive shaft 314 and the stylet tip 808 described above are symmetrical, e.g., both semi-circular. However, it will be appreciated that such symmetry may not be present. The sectional profiles may be differently shaped. For example, the shaft profile 602 may be a smaller or larger sector of a circle, e.g., a pie shape having rays separated by an angle of less than 180 degrees, and the tip profile 904 may be a complementary sector of the circle that, when engaged with the shaft profile 602, forms a full circle. Other shapes may be contemplated, which divide a circle into two, conforming profiles. For example, the mating shapes may include: a rectangular slot and key extending radially outward from a center of a circle, mating forms such as two interlocking, teardrop-shaped halves (e.g., an S-shaped dividing line similar to a yin and yang shape), etc. In any case, the shaft profile 602 and the tip profile 904 can mesh to fill the coil lumen 312 and permit torque transmission directly from the drive stylet 301 to the drive shaft 314. In each such case, the shaft profile 602 and the tip profile 904 are defined by outward-facing, outermost radial surfaces of the respective components, such that torque is transmitted through engagement of outward-facing surfaces, rather than through insertion of a drive tip into an inward-facing interior slot or cavity of the drive shaft 314.

A method of delivering torque to the drive shaft 314 by the drive stylet 301 is described below. The torque delivery may be used to drive the implantable lead 100 into a target tissue. Accordingly, at an operation, a delivery catheter can be navigated to the target tissue. For example, the delivery catheter can be tracked through a vein into a right ventricle and then a distal end of the delivery catheter can be positioned against a ventricular septal wall 106 having the target tissue. The delivery catheter may be of a type that is typically used for left bundle branch delivery.

At an operation, the implantable lead 100 can be deployed into the delivery catheter. The implantable lead 100 can be advanced through the delivery catheter to the target tissue. More particularly, the implantable lead 100 can be tracked through a delivery lumen of the delivery catheter until the helical fixation element 108 of the implantable lead 100 is flush with the distal end of the delivery catheter and/or the septal wall 106.

Referring to FIG. 12, a perspective view of an implantable lead system 300 having a drive stylet 301 inserting into an implantable lead 100 is shown in accordance with an embodiment. A proximal lead portion 1202 of the implantable lead 100 can include a seal 1204 and a connector pin 1206. The seal 1204 can be at a proximal end of the lead body 202, and can create a seal between the implantable lead 100 and a header of a biostimulator when the connector pin 1206 is plugged into an electrical port of the biostimulator. The connector pin 1206 can conduct electrical signals between the electrical port and the electrode(s) of the implantable lead 100. For example, the connector pin 1206 can be electrically connected to the inner coil 306 to conduct electrical signals to the helical fixation element 108 via the drive coupling 310. In an embodiment, the connector pin 1206 is rotatably mounted in the lead body 202 and can transmit torque to the inner coil 306 to rotate the drive coupling 310 for extending or retracting the helical fixation element 108 relative to the lead body 202.

In an embodiment, the connector pin 1206 can include a pin lumen 1208 extending along the central axis 204 into the lead body 202. More particularly, the pin lumen 1208 can extend through the connector pin 1206 into the coil lumen 312 of the inner coil 306 to provide a pathway to the drive shaft 314 of the drive coupling 310. For example, at an operation, the drive stylet 301 can be inserted into the pin lumen 1208 and advanced through the implantable lead 100 to engage the drive shaft 314 of the drive coupling 310.

As described above, the drive stylet 301 can extend along a central shaft axis from a proximal shaft end (not shown) to the distal shaft end. The drive stylet 301 can be an elongated member, e.g., a cylindrical wire, sized to slide freely within the pin lumen 1208 and the coil lumen 312 of the implantable lead 100. The drive stylet 301 can be formed from a metal or another material having a high torsional stiffness. Accordingly, an operator can twist the proximal shaft end to transmit torque through the drive stylet 301 to the stylet tip 808 at the distal shaft end. The stylet tip 808 can include the tip profile 904, which can engage the shaft profile 602 of the drive shaft 314, and the torque may therefore transfer through the drive stylet 301 to the drive coupling 310.

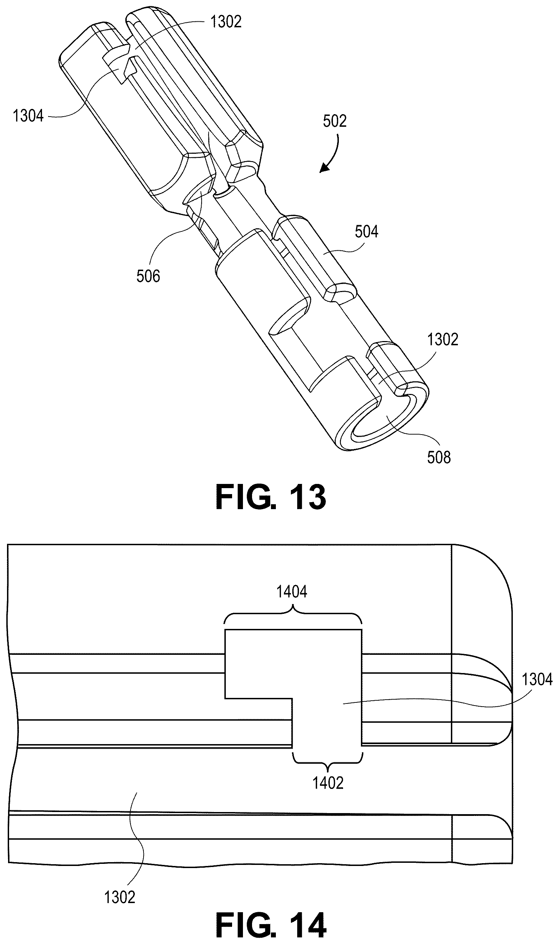

Referring to FIG. 13, a section view of an implantable lead system 300 having a drive stylet 301 disengaged from a drive coupling 310 of an implantable lead 100 is shown in accordance with an embodiment. A particular advantage of the implantable lead system 300 described herein includes a self-aligning characteristic of the drive stylet 301 and the drive shaft 314. The implantable lead system 300 can have a disengaged state in which the drive stylet 301 is not inserted into the complementary space 406 between the drive shaft 314 and the inner coil 306. The drive shaft 314 can include the shaft profile 602, which forms the complementary space 406 to receive the stylet tip 808. Accordingly, the drive shaft 314 can have one or more cavities or protrusions to receive torque from the stylet tip 808, e.g., a semicircular cavity to receive the stylet tip 808, and the stylet tip 808 can be proximally positioned outside of the cavity in the disengaged state.

In the disengaged state, the stylet tip 808 can be guided toward the complementary space 406 by the inner coil 306. For example, the inner coil surface 410 can provide a radially inward surface to define the coil lumen 312 through which the drive stylet 301 can travel toward the drive shaft 314. The drive stylet 301 is shown aligned with the complementary space 406, but proximal to the complementary space 406, in FIG. 13. It will be appreciated, however, that the drive stylet 301 may be misaligned with the drive shaft 314. More particularly, the stylet tip 808 may be out of alignment with the complementary space 406 such that the ends of the drive shaft 314 and the drive stylet 301 abut each other. In such case, the drive stylet 301 may not be engaged with the drive shaft 314. More particularly, at an operation, the physician can feed the drive stylet 301 through the implantable lead 100 until the stylet tip 808 is stopped at the drive shaft 314 end. The physician may rotate the drive stylet 301 to align the stylet tip 808 with the complementary space 406 and to advance the drive stylet 301 into locking engagement with the drive shaft 314. Alternatively, the physician may follow the procedure below, using a locking accessory, to cause the drive stylet 301 to passively and automatically align and engage with the drive shaft 314 without the need for active alignment of the components by the physician.

Referring to FIG. 14, a perspective view of a locking tool 1402 on a drive stylet 301 of an implantable lead system 300 is shown in accordance with an embodiment. At an operation, the physician attaches a locking tool 1402 to the connector pin 1206 on the proximal end of the implantable lead 100. The locking tool 1402 can be a component of the implantable lead system 300. For example, the locking tool 1402 can be packaged or used with the implantable lead 100 and the drive stylet 301. The locking tool 1402 can function to lock the implantable lead 100 to the drive stylet 301. More particularly, as described below, the locking tool 1402 can receive the lead body 202 and the drive stylet 301 to fix the lead body 202 relative to the drive stylet 301.

In an embodiment, the locking tool 1402 includes a tool body 1404. The tool body 1404 can have a distal section configured to grip the implantable lead 100. For example, the distal section can have an internal bore sized to press fit onto the seal 1204 or the connector pin 1206. More particularly, the tool body 1404 can have a body wall 1406 extending around a tool lumen 1408. The distal section can have a portion of the tool lumen 1408 sized to form a friction fit with a portion of the implantable lead 100. A proximal section of the locking tool 1402 can be mounted on the drive stylet 301. For example, the proximal section can have a portion of the tool lumen 1408 sized to form a friction fit with the proximal stylet portion 804 of the drive stylet 301.

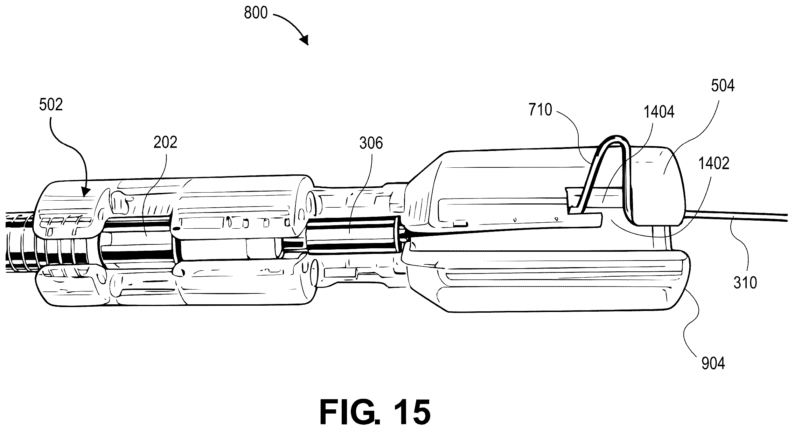

Referring to FIG. 15, a perspective view of a locking tool 1402 holding an implantable lead 100 and a drive stylet 301 of an implantable lead system 300 is shown in accordance with an embodiment. The drive stylet 301 can transmit torque directly to the drive shaft 314 and, thus, the helical fixation element 108. In some cases, however, it may be advantageous to lock the drive stylet 301 to the lead body 202 such that the implantable lead 100 and the drive stylet 301 operate as a unitary body. For example, when the helical fixation element 108 is not fixed to the lead body 202, e.g., is extendable, rotating the helical fixation element 108 by the drive stylet 301 may not rotate the lead body 202 and can limit device deliverability. Similarly, even when the helical fixation element 108 is fixed to the lead body 202, delivering torque directly to the drive shaft 314 without also rotating the proximal lead portion 1202 may cause wrinkling or torque buildup in the lead body 202, which can adversely affect deliverability. Accordingly, the locking tool 1402 can constrain or lock the drive stylet 301 relative to the lead body 202. More particularly, when the locking tool 1402 is mounted on the implantable lead 100 and the drive stylet 301, as shown, in FIG. 15, the locking tool 1402 receives the lead body 202 and the drive stylet 301 to fix the lead body 202 relative to the drive stylet 301 within the tool lumen 1408. The locking tool 1402 can constrain the drive stylet 301 relative to the lead body 202 both laterally relative to the central axis 204 and rotationally about the central axis 204.

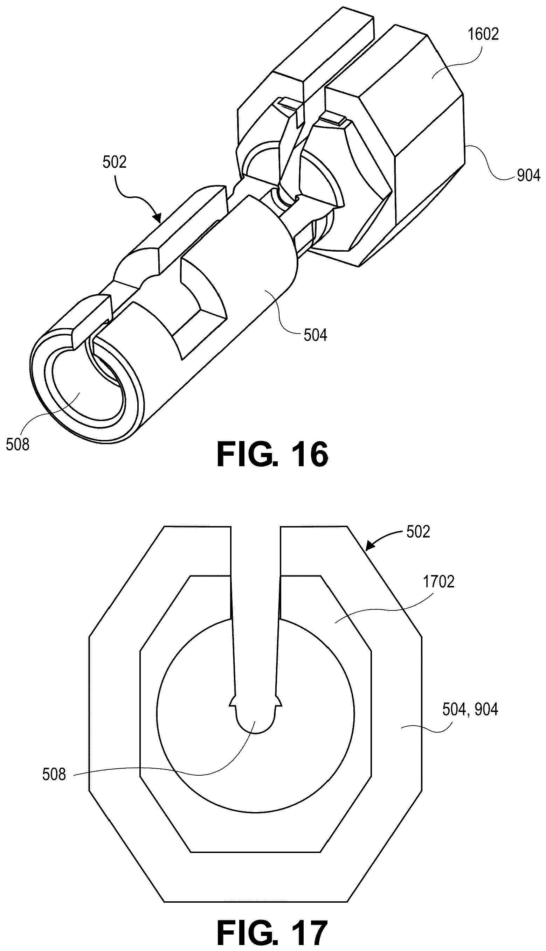

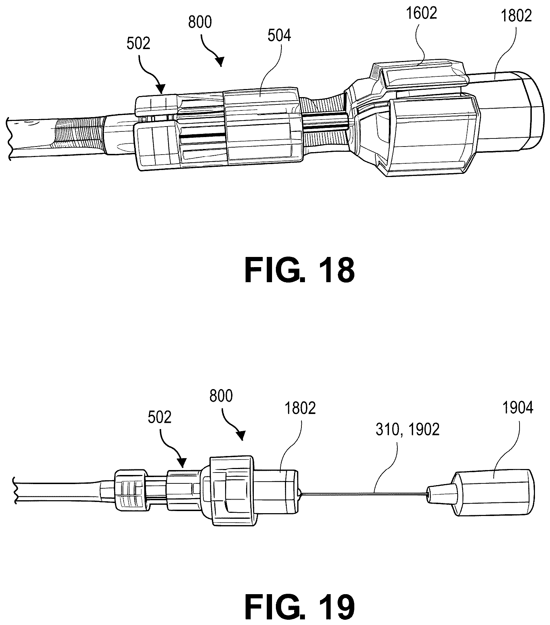

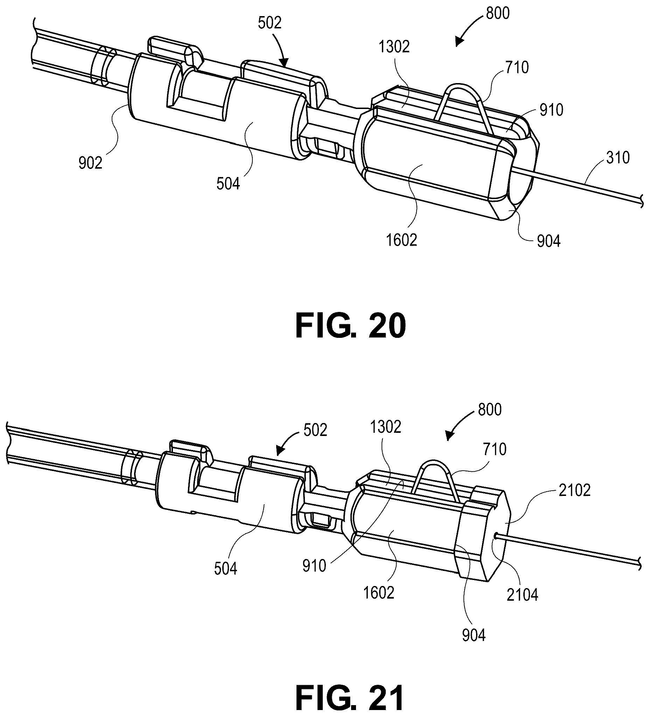

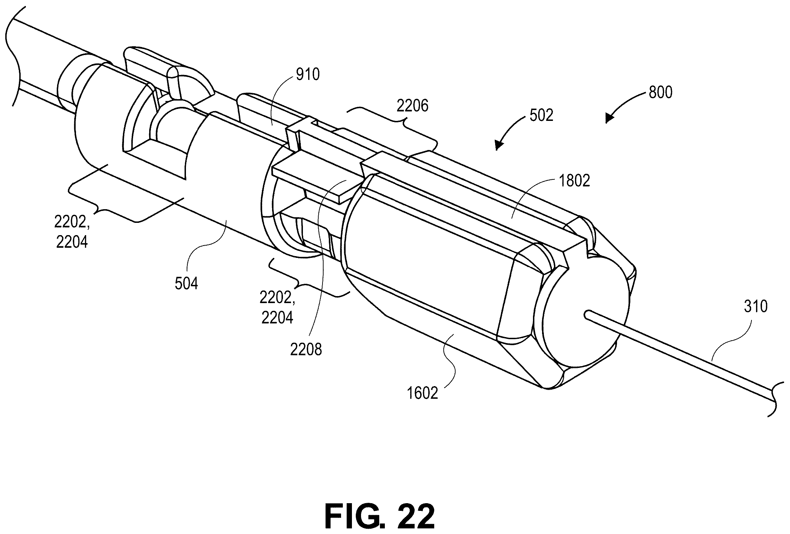

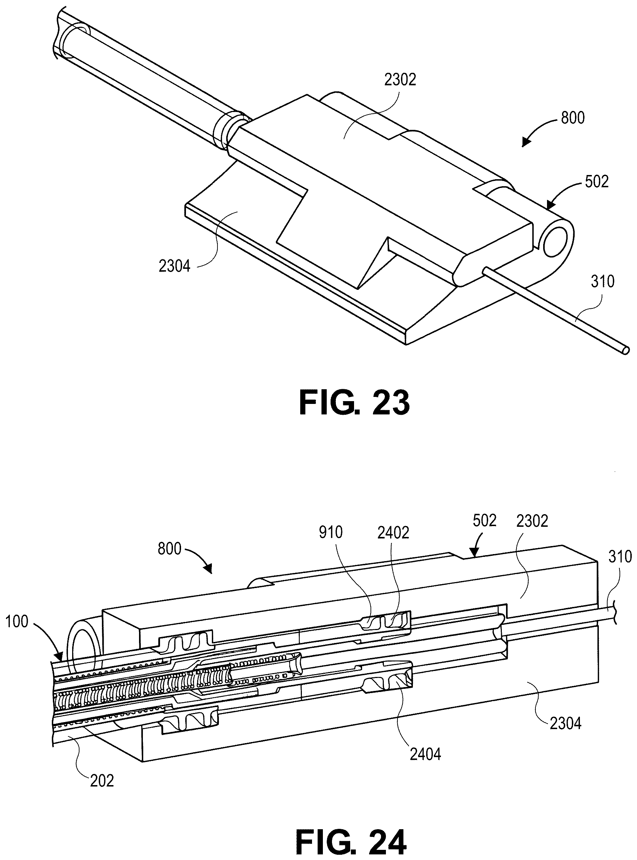

Referring to FIG. 16, a section view of an implantable lead system 300 having a drive stylet 301 engaged with a drive coupling 310 of an implantable lead 100 is shown in accordance with an embodiment. As described above, when the drive stylet 301 is initially inserted into the implantable lead 100, the stylet tip 808 may be misaligned and in contact with the drive shaft 314 (not engaged within the complementary space 406). Loading the locking tool 1402 onto the components can then lock the drive stylet 301 and implantable lead 100 together, causing the misaligned position to become persistent. Advantageously, however, it has been shown that the locking operation can result in, even when the components are misaligned, a distal force being placed on the implantable lead 100 by the drive stylet 301 that can place the lead body 202 in tension. More particularly, the lead body 202 can stretch slightly and/or apply a counterforce in a proximal direction on the stylet tip 808. As described below, this back force can ultimately cause the drive stylet 301 to passively and automatically align with the drive shaft 314.

At an operation, the physician can rotate the fixed implantable lead system 300 to drive the implantable lead 100 into the target tissue. The forward pressure applied by the locking tool 1402 assists the mating between the drive stylet 301 and the drive shaft 314. When the locking tool 1402 is rotated by the physician, with the drive stylet 301 pressed forward against the drive shaft 314 in misalignment, the elements of the implantable lead 100, e.g., the inner coil 306, the lead body 202 polymer layers, etc., will apply a restorative force to pull the drive shaft 314 into engagement with the drive stylet 301. More particularly, even when the proximal portions of the drive stylet 301 and implantable lead 100 are fixed to each other, relative differences in stiffness (e.g., the lead body 202 being less torsionally stiff than the drive stylet 301) can cause the distal ends of the components to rotate relative to each other. For example, the stylet tip 808 can rotate more than the distal portion of the lead body 202 when an input torque is applied, and the stylet tip 808 can therefore rotate into alignment with the complementary space 406. Furthermore, the tension in the lead body 202 and back force can draw the drive shaft 314 backward into engagement with the stylet tip 808, as shown in FIG. 16, when the stylet tip 808 aligns with the complementary space 406. More particularly, the stylet tip 808 can engage the drive shaft 314.

Referring to FIG. 17, a perspective view of a locking tool 1402 rotating an implantable lead system 300 is shown in accordance with an embodiment. When the stylet tip 808 is within the complementary space 406 in a mating relationship with the implantable lead 100, the system is in the engaged state. In the engaged state, torque can be transferred from the drive stylet 301 to the distal end of the implantable lead 100. It will be appreciated that the self-alignment of the stylet tip 808 to the drive shaft 314 is passive and automatic, not requiring the physician to attempt to actively align the components and, thus, can simplify the procedure used by the physician. More particularly, the physician can rotate the locking tool 1402 to torque the implantable lead 100. Alternatively, the physician can rotate a knob 1702, which is mounted on the proximal stylet portion 804 of the stylet body 805, to transmit torque through the drive stylet 301. In either case, the physician can torque the implantable lead 100 into place using a direct torque transmission without having to actively try to align the drive components.

An aspect of the embodiments relates to an implantable lead 100. The implantable lead 100 comprises a lead body 202 extending along a central axis 204 and having a central lumen 208. The implantable lead 100 also comprises a drive coupling 310 rotatably mounted in the central lumen 208. The drive coupling 310 includes a drive shaft 314 having an outer shaft surface 402 defining a shaft profile 602. The shaft profile 602 is non-circular. The implantable lead 100 further comprises a helical fixation element 108 mounted on the drive coupling 310.

In an embodiment, the shaft profile 602 has a shaft circular arc 604 extending about the central axis 204 between a first shaft edge 502 and a second shaft edge 504, and a shaft non-circular segment 606 extending between the first shaft edge 502 and the second shaft edge 504.

In an embodiment, the shaft profile 602 includes a semicircular segment 608.

In an embodiment, the drive shaft 314 includes a transitional edge 702 between a proximal shaft end 704 and a shaft drive surface 408.

In an embodiment, the implantable lead 100 further comprises an inner coil 306 extending through the lead body 202 along the central axis 204. The inner coil 306 is coupled to the drive shaft 314.

In an embodiment, the drive shaft 314 is disposed in a coil lumen 312 of the inner coil 306 such that a complementary space 406 is disposed between a shaft drive surface 408 of the drive shaft 314 and an inner coil surface 410 of the inner coil 306.

In an embodiment, the implantable lead 100 further comprises a joint 420 between the inner coil 306 and the drive shaft 314.

In an embodiment, the outer shaft surface 402 of the drive shaft 314 includes a stepped surface 404. The joint 420 is between a distal coil end of the inner coil 306 and the stepped surface 404.

Another aspect of the embodiments relates to a drive stylet 301. The drive stylet 301 comprises a stylet body 805 extending along a central axis 204 from a proximal stylet portion 804 to a distal stylet portion 806. The stylet body 805 includes a stylet tip 808 at the distal stylet portion 806. The stylet tip 808 includes an outer tip surface 902 defining a tip profile 904. The tip profile 904 is non-circular, and a knob 1702 mounted on the proximal stylet portion 804 of the stylet body 805.

In an embodiment, the tip profile 904 is non-circular to engage the non-circular shaft profile 602 of a drive shaft as defined above.

In an embodiment, the tip profile 904 has a tip circular arc 1006 extending about the central axis 204 between a first tip edge 1002 and a second tip edge 1004, and a tip non-circular segment 1008 extending between the first tip edge 1002 and the second tip edge 1004.

In an embodiment, the tip profile 902 includes a semicircular segment 1005.

A further aspect of the embodiments relates to an implantable lead system 300. The implantable lead system 300 comprises an implantable lead 100 including a lead body 202, a drive coupling 310 rotatably mounted in a central lumen 208 of the lead body 202, and a helical fixation element 108 mounted on the drive coupling 310. The lead body 202 extends along a central axis 204. The drive coupling 310 includes a drive shaft 314 having an outer shaft surface 402 defining a shaft profile 602. The shaft profile 602 is non-circular. The implantable lead system 300 also comprises a drive stylet 301 including a stylet body 805 and a knob 1702 mounted on a proximal stylet portion 804 of the stylet body 805. The stylet body 805 extends along the central axis 204 from the proximal stylet portion 804 to a distal stylet portion 806. The stylet body 805 includes a stylet tip 808 at the distal stylet portion 806. The stylet tip 808 includes an outer tip surface 902 defining a tip profile 904. The tip profile 904 is non-circular.

In an embodiment, the implantable lead system 300 comprises the implantable lead 100 as defined above and the drive stylet as defined above.

In an embodiment, the stylet tip 808 is configured to engage the drive shaft 314 such that the outer shaft surface 402 and the outer tip surface 902 are complementary to form a circular outer profile 1102.

In an embodiment, the drive shaft 314 includes a transitional edge 702 between a proximal shaft end 704 and a shaft drive surface 408.

In an embodiment, the shaft profile 602 includes a shaft circular arc 604 extending about the central axis 204 between a first shaft edge 502 and a second shaft edge 504, and a shaft non-circular segment 606 extending between the first shaft edge 502 and the second shaft edge 504.

In an embodiment, the shaft profile 602 includes a semicircular segment 608.

In an embodiment, the implantable lead system 300 further comprises an inner coil 306 extending through the lead body 202 along the central axis 204. The inner coil 306 is coupled to the drive shaft 314.

In an embodiment, the tip profile 904 includes a tip circular arc 1006 extending about the central axis 204 between a first tip edge 1002 and a second tip edge 1004, and a tip non-circular segment 1008 extending between the first tip edge 1002 and the second tip edge 1004.

In an embodiment, the tip profile 904 includes a semicircular segment 1005.

In an embodiment, the implantable lead system 300 further comprises a locking tool 1402 including a tool body 1404 having a body wall 1406 extending around a tool lumen 1408.

In an embodiment, the locking tool 1402 is configured to receive the lead body 202 and the drive stylet 301 to fix the lead body 202 relative to the drive stylet 301 within the tool lumen 1408.

In the foregoing specification, the invention has been described with reference to specific exemplary embodiments thereof. It will be evident that various modifications may be made thereto without departing from the broader spirit and scope of the invention as set forth in the following claims. The specification and drawings are, accordingly, to be regarded in an illustrative sense rather than a restrictive sense.

## Claims

1. An implantable lead (100), comprising:
a lead body (202) extending along a central axis (204) and having a central lumen (208);
a drive coupling (310) rotatably mounted in the central lumen (208), wherein the drive coupling (310) includes a drive shaft (314) having an outer shaft surface (402) defining a shaft profile (602), and wherein the shaft profile (602) is non-circular; and
a helical fixation element (108) mounted on the drive coupling (310).

2. The implantable lead of claim 1, wherein the shaft profile (602) has a shaft circular arc (604) extending about the central axis (204) between a first shaft edge (502) and a second shaft edge (504), and a shaft non-circular segment (606) extending between the first shaft edge (502) and the second shaft edge (504).

3. The implantable lead of claim 2, wherein the shaft profile (602) includes a semicircular segment (608).

4. The implantable lead of any one of claims 1 to 3, wherein the drive shaft (314) includes a transitional edge (702) between a proximal shaft end (704) and a shaft drive surface (408).

5. The implantable lead of any one of claims 1 to 4, further comprising an inner coil (306) extending through the lead body (202) along the central axis (204), wherein the inner coil (306) is coupled to the drive shaft (314).

6. The implantable lead of claim 5, wherein the drive shaft (314) is disposed in a coil lumen (312) of the inner coil (306) such that a complementary space (406) is disposed between a shaft drive surface (408) of the drive shaft (314) and an inner coil surface (410) of the inner coil (306).

7. The implantable lead of claim 5 or 6, further comprising a joint (420) between the inner coil (306) and the drive shaft (314).

8. The implantable lead of claim 7, wherein the outer shaft surface (402) of the drive shaft (314) includes a stepped surface (404), and wherein the joint (420) is between a distal coil end of the inner coil (306) and the stepped surface (404).

9. A drive stylet (301), comprising:
a stylet body (805) extending along a central axis (204) from a proximal stylet portion (804) to a distal stylet portion (806), wherein the stylet body (805) includes a stylet tip (808) at the distal stylet portion (806), wherein the stylet tip (808) includes an outer tip surface (902) defining a tip profile (904), and wherein the tip profile (904) is non-circular; and
a knob (1702) mounted on the proximal stylet portion (804) of the stylet body (805).

10. The drive stylet of claim 9, wherein the tip profile (904) has a tip circular arc (1006) extending about the central axis (204) between a first tip edge (1002) and a second tip edge (1004), and a tip non-circular segment (1008) extending between the first tip edge (1002) and the second tip edge (1004).

11. The drive stylet of claim 10, wherein the tip profile (904) includes a semicircular segment (1005).

12. An implantable lead system (300), comprising:
an implantable lead (100) according to any one of claims 1 to 8; and
a drive stylet (301) according to any one of claims 9 to 11.

13. The implantable lead system of claim 12, wherein the stylet tip (808) is configured to engage the drive shaft (314) such that the outer shaft surface (402) and the outer tip surface (902) are complementary to form a circular outer profile (1102).

14. The implantable lead system of claim 12 or 13, further comprising a locking tool (1402) including a tool body (1404) having a body wall (1406) extending around a tool lumen (1408).

15. The implantable lead system of claim 14, wherein the locking tool (1402) is configured to receive the lead body (202) and the drive stylet (301) to fix the lead body (202) relative to the drive stylet (301) within the tool lumen (1408).
